# EUROPEAN PATENT SPECIFICATION

(11) **EP 0 946 139 B1**
(45) Date of publication and mention of the grant of the patent: **05.06.2002**
(21) Application number: 97944000.5
(22) Date of filing: 14.10.1997
(51) Int. Cl.: A61K 7/48, A61K 7/50

(54) **PHARMACEUTICAL FORMULATION**
PHARMAZEUTISCHE FORMULIERUNG
COMPOSITION PHARMACEUTIQUE

(30) Priority: 15.10.1996 GB 9621480
(43) Date of publication of application: 06.10.1999
(73) Proprietor: Crawford Healthcare Ltd., Milton Keynes MK19 6AQ (GB)
(72) Inventor: MORLEY, Robin, Milton Keynes MK19 6AQ (GB); BEDFORD-STRADLING, Michael, Milton Keynes MK19 6AQ (GB)
(74) Representative: Sanderson, Laurence Andrew
(86) International application number: GB9702716
(87) International publication number: WO9816195

(56) References cited:
- EP-A- 0 039 874
- GB-A- 2 052 980
- US-A- 4 983 625

## Description

The present invention relates to pharmaceutical formulations comprising 8-methoxypsoralen; in particular, those suitable for use as a bath additive.

8-Methoxypsoralen (known as 8-MOP) is a furocoumarin extracted from the Amni Majus plant which grows in India and Egypt. This substance has been used for many hundreds of years to treat vitiligo since it is a powerful photo-sensitising agent, and in conjunction with ultra violet light of a certain wavelength, such as on a wavelength of about 365 nanometres, induces a tanning effect on the skin as well as altering DNA synthesis.

Some oral preparations have been increasingly used by dermatologists in specialised units to treat a variety of dermatoses. Oral treatment using 10mg 8-MOP in a starch/lactose tablet, whilst effective, causes a number of side effects (e.g. nausea and headaches) with certain patients. Paint and emulsion formulations are ideal for the treatment of small areas of the body but could not be practically used for whole body treatment.

There was therefore a need for a formulation that could be used as a bath oil to facilitate whole body treatment and avoid the side effects caused by oral therapy. Some dermatologists were already familiar with this form of treatment since trimethylpsoralen (T.M.P.), a similar furocoumarin, dissolved in an alcoholic base, was being used in Scandinavia. However, alcohol and water phases do not mix readily in a bath solution.

The specific need is therefore for a bath additive formulation based on an aqueous (non-alcoholic) solution that could be diluted in the bath and would be acceptable to doctors and patients alike. 8-MOP is known to be practically insoluble in water, sparingly soluble in boiling water and freely soluble in a number of organic solvents such as chloroform, acetone, acetic acid and propylene glycol.

British Patent Publication No. 2,052,980 discloses a pharmaceutical formulation comprising 8-methexy-psoralen. However, the formulation taught in this reference is intended for oral administration, and is not suitable for addition to bath water for whole body topical application.

US patent specification no. 4,983,625 discloses a bath composition for use in the treatment of, *inter alia,* psoriasis, said composition being provided in the form of an oily product and comprising 10-79.1% w/w of a hydrophilic phase, 20-89.9% w/w of a lipophilic or fatty phase, and 0.1-2% w/w of a psoralene. However, after mixing these bath compositions with bath water, the bath is said to have a homogeneous milky appearance. Such compositions have therefore not been commercialised since there is a recognised danger from the use of emulsions during treatment with UV light. In practice, although the emulsion may at first be monogeneous, it soon concentrates the 8-MOP in certain areas of the bathwater such as at the surface around the patient's body. Such a high concentration close to the skin gives rise to the danger of burning on exposure to UV rays. The only practical way for the patient to avoid this side-effect would be to keep moving about in the bath or otherwise `stirring' the bathwater.

There is therefore the need for a bath additive containing 8-MOP, preferably at a concentration of approximately 1%, which forms a clear, homogeneous solution when added to the bath water and which remains stable under a variety of storage conditions.

The present invention therefore provides a substantially anhydrous topical pharmaceutical formulation suitable for use as a bath additive comprising 8-methoxypsoralen and a pharmacologically-acceptable carrier therefor, which carrier comprises:
(a) a pharmacologically-acceptable hydrophilic solubilising agent for the 8-methoxypsoralen;
(b) a non-ionic, water-in-oil emulsifier; and, preferably
(c) a clarifying agent; and, optionally
(d) an anhydrous detergent.

Preferably, the concentration of 8-MOP in the formulation is in the range of from 1.1 to 1.3%, such as 1.170 to 1.293%, preferably about 1.2% w/v. At 20°C, the pH of the formulation is preferably mildly acidic, with the pH of a 1% w/v formulation being in the range of from 5 to 7, such as 5.5 to 6.5, preferably about 6.5.

Component (a) is a solubiliser for the 8-MOP which must also be soluble in water. The solubiliser is preferably a water-dispersible oil, and is more preferably a compound which acts as a superfatting agent. Such solubilisers should also be pharmacologically and/or cosmetically acceptable in the sense of not causing any adverse reactions to skin submerged in the diluted formulation. Suitable solubilisers are therefore non-ionic, emollient, superfatting oils. Preferred solubilisers are selected from polyol fatty acid esters for example those having a hydrophile-lipophile balance (HLB) in the range of about 11 to about 13 such as PEG-7 glyceryl cocoate which is available, inter alia, under the trade name Cetiol HE from Henkel KGaA, Dusseldorf, Germany. Preferably, component (a) is present in an amount in the range of from 15-60% w/w, more preferably 20-40% w/w, especially about 32-38% w/w.

Preferably, a component (b) comprises a non-ionic water-in-oil emulsifier such as a polyethylene glycol ether, for example one selected from C₁₂₋₁₈ saturated or unsaturated fatty alcohols having from 3 to 10 moles of ethylene oxide per molecule. For example, Oleth-10, the polyethylene glycol ether of oleyl alcohol having 10 moles of ethylene oxide is available under the trade name Brij 97. A particularly preferred emulsifier is laureth-3, available from Henkel (q.v.) under the registered trade mark Dehydol LS3 DO and comprising C₁₂₋₁₄ saturated fatty alcohols having 3 moles of ethylene oxide per molecule. Preferably, from about 15 to about 50% w/w is added to the formulation, more preferably in the range of from 20-40% w/w, especially about 30% w/w. Component (b) may have a HLB in the range of about 7.5 to about 12.5.

In particular to assist with clarity of the final formulation in the bath water, component (c) may be selected from non-ionic water-in-oil emulsifiers which are preferably polymers and more preferably ethoxylated polymers. For example, component (c) may be selected from fatty acid esters of polyethylene glycols such as esters of castor oil fatty acids with ethoxylated glycerine; and fatty acid esters of polyethylene glycol having about 20 moles of ethylene oxide per molecule such as wherein the fatty acid ester is a mixture of esters of sorbitol and sorbitol anhydrides, for example, C₁₂₋₁₈ saturated or unsaturated fatty acids such as lauric or oleic acids. Accordingly, suitable esters include polysorbate 20 arid polysorbate 80 available, inter alia, under the trade names Tween 20 and Tween 80, respectively. Another example of a suitable non-ionic emulsifier is a polyethylene glycol ester of castor oil fatty acids with ethoxylated glycerine such as PEG-40 castor oil, which is available from Henkel (q.v.) under the registered trade mark Eumulgin RO 40. Preferably, component (c) is present in an amount in the range of from 10-40% w/w, more preferably 15-25% w/w, especially about 20% w/w.

Since they are anhydrous, the formulations of the present invention are substantially free from water. Preferably, the formulations of the present invention are also substantially free from monomeric fatty acids, esters or alcohols, hydrocarbon oils such as petrolatum and mono-, di- or triglycerides of fatty acids or vegetable oils.

Preferably, other excipients which are liquid at room temperature are added to assist with emulsification and use, particularly in terms of clarity of the final bath water, ease of draining the bath water and reduction of oily film residue on the bath, once emptied.

Other ingredients which may assist in emulsification and which form substantially clear dispersions in bath water include substantially anhydrous detergents (component (d)) having low viscosity of the order of less than 1 mPa at 10°C such as mixtures of anionic detergents such as alkyl ether sulphates based on coconut oil with non-ionic emulsifiers. For example, MIPA-laureth sulphate (anionic detergent) and cocamide DEA (non-ionic foam stabiliser) mixtures optionally mixed with laureth-3 are effective as wetting agents and ensure that the bath water can be drained away without leaving an oily residue. Such mixtures also have good refatting properties, ensuring that the patient's skin is not dried out after soaking in the formulation according to the present invention. A preferred combination wetting agent is sold under the registered trade mark Texapon WW 99 and is available from Henkel (q.v.). Preferably, component (d) is present in an amount in the range of from 10-30% w/w, more preferably 10-20% w/w, especially about 15% w/w.

Preferably, the total weight of the formulation per ml is in the range of from about 1.00 to 1.02 g/ml at 20°C, such as about 1.005 g/ml. The total weight of the solids in the formulation preferably comprises from about 67.5 to 69.5%, such as about 69.8%.

As is appreciated by the person skilled in the art, all percentage weights given herein are subject to a tolerance of + or -5%.

Preferably, the present invention provides a process wherein 8-MOP is dissolved in the solubiliser component (a), more preferably with mild heating (about 60°C). The remaining ingredients are then added, preferably with stirring and preferably with the temperature maintained at about 60°C. More preferably, the non-ionic emulsifiers are added together (components (b) and (c)), followed by the wetting agent (component (d)). Preferably, stirring is then continued while the temperature of the mixture is cooled to below 30°C. The final formulation is then packaged such as in amber tamper-proof bottles. Preferably, the bottles are of unit dose size and therefore the bottles preferably contain 30ml of solution. For semi-bulk supply, 12 such bottles may be packed into labelled, cardboard packaging.

The formulation of the present invention is indicated for the treatment of various skin disorders. The methoxypsoralen concentrates in the melanocytes and absorbs ultraviolet energy, thus promoting the production of melanine pigmentry in the vitiligenous areas. Ultraviolet radiation can be either from natural means or from an ultra violet lamp. The formulation is also useful in the treatment of psoriasis, in which case it is recommended that the patient should be treated by means of artificial ultraviolet radiation preferably on a wavelength of about 365 nanometres.

In use, the formulation of the present invention is preferably added to a bath filled with warm (50-60°C) water. Preferably, the concentration of 8-MOP in the bath is in the range of from about 1.8 mg/l to about 3.8 mg/l. For example, in a bath filled with water up to the 140l/30gall mark, 33ml of the formulation lotion is added to produce a final concentration of 8-MOP of about 2.6mg/l. The patient lies in the water and sluices themselves for about 15 minutes, taking care to avoid contact with the eyes. Immediately after drying with a towel, patients receive an appropriate dose of UVA (wavelength 365 nanometres), dosage dependent on the patient. Bath water delivery of 8-methoxypsoralen has been found to be as effective as oral administration of 8-methoxypsoralen and yet requires smaller amounts of ultraviolet radiation and yields fewer side effects.

The present invention will now be illustrated by the following nonlimiting examples.

| Example 1: 8-MOP Bath Lotion - Bulk Manufacture | |
|---|---|
| 8-Methoxypsoralen (8-MOP) | 1176.0g |
| Dehydol LS3 | 31500.0g |
| Cetiol HE | 35700.0g |
| Eumulgin RO40 | 21000.0g |
| Texapon WW99 | 15624.0g |

Using a Silverson mixer, fitted with an emulsor head at high speed, methoxsalen is dissolved, in a 6001 jacketed stainless steel tank, in Cetiol HE (registered trade mark) with. the aid of heat (not above 65°C). The air is allowed to come out of the solution, then Dehydol D53 and Eumulgin RO40 are added at the same temperature, and the mixture stirred well. Then Texapon WW99 is added, ensuring it is at the same temperature as the bulk and stirred well. Stirring is continued until the temperature drops below 30°C. The bulk is left to stand for 30 minutes, then a 20ml sample is taken from the top and bottom of the bulk (to ensure no separation of the components and that the methoxsalen is uniformly distributed). The bulk is stirred again for a further 15 minutes.

| Example 2: 8-MOP Bath Lotion - 100g Batches | |
|---|---|
| 8-MOP | 1.12g |
| Dehydol LS3 | 30.0g |
| Cetiol HE | 34.0g |
| Eumulgin RO40 | 20.0g |
| Texapon WW99 | 14.9g |

All the required ingredients were weighed out. The Cetiol was heated to 60-65° in a water bath, then the 8-MOP was added and mixed using a Silverson mixer at high speed until all the drug had dissolved. Where possible, all the entrapped air was allowed to come out of the solution before proceeding. The Dehydol and Eumulgin were then added, with vigorous stirring. The Texapon was then slowly added, and this was allowed to cool to 30° with gentle mixing. The final product was placed into 30ml containers.

## Claims

1. A substantially anhydrous pharmaceutical bath-additive formulation comprising 8-methoxypsoralen (8-MOP) and a pharmacologically-acceptable carrier therefor adapted on addition to bath-water for forming a clear homogeneous solution for whole-body topical application, which carrier comprises:
(a) a pharmacologically-acceptable hydrophilic solubilising agent for the 8-methoxypsoralen; and
(b) a non-ionic, water-in-oil emulsifier;
as well as optionally
(c) a clarifying agent; and/or
(d) an anhydrous detergent.

2. A bath-additive formulation as claimed in claim 1, in which the carrier includes a clarifying agent (c) selected from non-ionic, water-in-oil polymeric emulsifiers.

3. A bath-additive formulation as claimed in claim 2, in which the clarifying agent (c) is or includes ethoxylated polymer(s).

4. A bath-additive formulation as claimed in claim 1, in which the carrier includes a clarifying agent (c) selected from fatty acid esters of polyethylene glycols.

5. A bath-additive formulation as claimed in claim 4, in which the clarifying agent (c) is or includes a polyethylene glycol ester of castor oil fatty acids with glycerine.

6. A bath-additive formulation as claimed in any of the preceding claims, in which the carrier includes an anhydrous detergent (d) selected from substantially anhydrous detergents having low viscosity of the order of less than 1mPa at 10°C.

7. A bath-additive formulation as claimed in any of the preceding claims which is substantially free from monomeric fatty acids, esters and alcohols, hydrocarbon oils, vegetable oils and mono-, di- and triglycerides of fatty acids.

8. A bath-additive formulation as claimed in any of the preceding claims, in which the carrier comprises:
(a) PEG 7 glyceryl cocoate; and
(b) Oleth-10 and/or Laureth-3;
(c) as well as optionally Polysorbate 20, polysorbate 80 and/or PEG-40 castor oil;
and/or optionally,
(d) Monoisopropanolamine (MIPA)-laureth sulphate and/or cocamide diethanolamide (DEA).

9. A bath-additive formulation as claimed in any the preceding claims 1 to 8, when adapted for use in the treatment of skin disorders such as psoriasis.

10. A bath-additive formulation as claimed in any of the preceding claims, which on addition to bath water in an amount that results in a concentration of 8-MOP in the bath water within the range of from 1.8 to 3.8mg per litre of bath water, yields bath water of homogeneously clear appearance.

## Patentansprüche

1. Im Wesentlichen wasserfreie, pharmazeutische Badezusatzformulierung, die 8-Methoxypsoralen (8-MOP) und einen pharmazeutisch verträglichen Träger dafür umfasst, welcher für die Zugabe zu Badewasser zur Ausbildung einer klaren homogenen Lösung für eine topische Applikation am ganzen Körper angepaßt ist, wobei der Träger umfasst:
(a) einen pharmazeutisch verträglichen hydrophilen Lösungsvermittler für 8-Methoxypsoralen; und
(b) einen nicht-ionischen Wasser-in-Öl-Emulgator;
sowie gegebenenfalls
(c) ein Klärungsmittel; und/oder
(d) ein wasserfreies Detergens.

2. Badezusatzformulierung gemäß Anspruch 1, in der der Träger ein Klärungsmittel (c) umfasst, das aus nicht-ionischen, polymeren Wasser-in-Öl Emulgatoren ausgewählt wurde.

3. Badezusatzformulierung gemäß Anspruch 2, in der das Klärungsmittel (c) ethoxylierte(s) Polymer(e) ist oder umfasst.

4. Badezusatzformulierung gemäß Anspruch 1, in der der Träger ein Klärungsmittel (c) umfasst, das aus Fettsäureestern von Polyethylenglykol ausgewählt wurde.

5. Badezusatzformulierung gemäß Anspruch 4, in der das Klärungsmittel (c) ein Polyethylenglykolester der Castorölfettsäuren mit Glycerin ist oder umfasst.

6. Badezusatzformulierung gemäß irgendeinem der vorstehenden Ansprüche, in der der Träger wasserfreies Detergens (d) umfasst, das aus im Wesentlichen wasserfreien Detergenzien ausgewählt wurde, welche eine geringe Viskosität im Bereich von weniger als 1 mPa bei 10 °C aufweisen.

7. Badezusatzformulierung gemäß irgendeinem der vorstehenden Ansprüche, die im Wesentlichen frei von monomeren Fettsäuren, Estern und Alkoholen, Kohlenwasserstoffölen, vegetabilen Ölen und Mono-, Di- und Triglyceriden der Fettsäuren ist.

8. Badezusatzformulierung gemäß irgendeinem der vorstehenden Ansprüche, in der der Träger Folgendes umfasst;
(a) PEG-7-glycerylcocoat; und
(b) Oleth-10 und/oder Laureth-3;
(c) sowie gegebenenfalls Polysorbat 20, Polysorbat 80 und/oder PEG-40-Castoröl; und/oder gegebenenfalls
(d) Monoisopropanolamin (MIPA)-Laurethsulfat und/oder Cocamiddiethanolamid (DEA).

9. Badezusatzformulierung gemäß irgendeinem der vorstehenden Ansprüche 1 bis 8, sofern diese zur Verwendung für der Behandlung von Hautstörungen, wie Psoriasis, angepaßt wurde.

10. Badezusatzformulierung gemäß irgendeinem der vorstehenden Ansprüche, die nach Zugabe zu Badewasser in einer Menge, die in einer Konzentration von 8-MOP von 1,8 bis 3,8 mg/l Badewasser im Badewasser resultiert, ein Badewasser mit homogener, klarer Erscheinung erzeugt.

## Revendications

1. Formulation pharmaceutique essentiellement anhydre d'additif pour le bain comprenant du 8-méthoxypsoralène (8-MOP) et un véhicule pharmacologiquement acceptable, adapté à l'addition à l'eau du bain pour la formation d'une solution homogène limpide pour une application topique sur tout le corps, lequel véhicule comprend :
(a) un agent de solubilisation hydrophile pharmacologiquement acceptable pour le 8-méthoxypsoralène ; et
(b) un émulsifiant eau dans l'huile, non ionique;
ainsi qu'éventuellement
(c) un agent de clarification; et/ou
(d) un détergent anhydre.

2. Formulation d'additif pour le bain selon la revendication 1, dans laquelle le véhicule comprend un agent de clarification (c) choisi parmi des émulsifiants polymériques eau dans l'huile, non ioniques.

3. Formulation d'additif pour le bain selon la revendication 2, dans laquelle l'agent de clarification (c) est ou comprend un (ou des) polymère(s) éthoxylé(s).

4. Formulation d'additif pour le bain selon la revendication 1, dans laquelle le véhicule comprend un agent de clarification (c) choisi parmi les esters d'acides gras des glycols polyéthyléniques.

5. Formulation d'additif pour le bain selon la revendication 4, dans laquelle l'agent de clarification (c) est ou comprend un ester de glycol polyéthylénique d'acides gras d'huile de ricin avec de la glycérine.

6. Formulation d'additif pour le bain selon l'une quelconque des revendications précédentes, dans laquelle le véhicule comprend un détergent anhydre (d) choisi parmi des détergents essentiellement anhydres ayant une faible viscosité de l'ordre de moins de 1 mPa à 10°C.

7. Formulation d'additif pour le bain selon l'une quelconque des revendications précédentes, qui est esentiellement exempte d'alcools, d'esters et d'acides gras monomériques, d'huiles hydrocarbonées, d'huiles végétales, et de mono-, di- et triglycérides d'acides gras.

8. Formulation d'additif pour le bain selon l'une quelconque des revendications précédentes, dans laquelle le véhicule comprend :
(a) du cocoate de glycéryl PEG 7 ; et
(b) de l'oleth-10 et/ou du laureth-3;
(c) ainsi qu'éventuellement du Polysorbate 20, du polysorbate 80 et/ou de l'huile de ricin PEG-40;
et/ou éventuellement,
(d) du sulfate de monoisopropanolamine (MIPA)-laureth et/ou du cocamide diéthanolamide (DEA).

9. Formulation d'additif pour le bain selon l'une quelconque des revendications précédentes 1 à 8, adaptée pour une utilisation dans le cadre du traitement de maladies de la peau telles que le psoriasis.

10. Formulation d'additifs pour le bain selon l'une quelconque des revendications précédentes, qui, à l'addition à l'eau de bain dans une quantité qui résulte en une concentration de 8-MOP dans l'eau du bain dans le domaine de 1,8 à 3,8 mg par litre d'eau de bain, donne une eau de bain d'apparence limpide et homogène.
